# EUROPEAN PATENT SPECIFICATION

(11) **EP 4 458 380 B1**
(45) Date of publication and mention of the grant of the patent: **23.09.2026**
(21) Application number: 22913811.0
(22) Date of filing: 08.11.2022
(51) Int. Cl.: A61L 27/08, A61L 27/18, A61B 5/00, A61L 27/50, A61L 27/30

(54) **CFR-PEEK ORTHOPEDIC IMPLANT AND PREPARATION METHOD THEREFOR, AND WIRELESS SENSING DEVICE**
ORTHOPÄDISCHES CFR-PEEK-IMPLANTAT UND HERSTELLUNGSVERFAHREN DAFÜR SOWIE DRAHTLOSE ERFASSUNGSVORRICHTUNG
IMPLANT ORTHOPÉDIQUE CFR-PEEK ET SON PROCÉDÉ DE PRÉPARATION, ET DISPOSITIF DE DÉTECTION SANS FIL

(30) Priority: 27.12.2021 CN 202111618690
(43) Date of publication of application: 06.11.2024
(73) Proprietor: Tsinghua University, Haidian District Beijing 100084 (CN)
(72) Inventor: ZANG, Xining, Beijing 100084 (CN); ZHAO, Zhe, Beijing 100084 (CN); HU, Xingjian, Beijing 100084 (CN); HUANG, Jincai, Beijing 100084 (CN); LI, Qingang, Beijing 100084 (CN)
(74) Representative: Dehns
(86) International application number: PCT/CN2022/130564
(87) International publication number: WO 2023/124548

(56) References cited:
- CN-A- 101 291 634
- CN-A- 108 007 480
- CN-A- 113 209 389
- CN-A- 114 432 491
- CN-U- 213 850 988
- US-A1- 2017 020 402
- US-A1- 2018 353 219
- DU YA-WEI ET AL: "Physical modification of polyetheretherketone for orthopedic implants", FRONTIERS OF MATERIALS SCIENCE, HIGHER EDUCATION PRESS, BEIJING, vol. 8, no. 4, 21 October 2014 (2014-10-21), pages 313 - 324, XP035410126, ISSN: 2095-025X, [retrieved on 20141021], DOI: 10.1007/S11706-014-0266-4
- HU XINGJIAN ET AL: "Laser Direct-Write Sensors on Carbon-Fiber-Reinforced Poly-Ether-Ether-Ketone for Smart Orthopedic Implants", vol. 9, no. 11, 10 February 2022 (2022-02-10), Germany, XP093332123, ISSN: 2198-3844, Retrieved from the Internet <URL:https://onlinelibrary.wiley.com/doi/full-xml/10.1002/advs.202105499> [retrieved on 20251104], DOI: 10.1002/advs.202105499
- HU XINGJIAN ET AL: "Laser Direct-Write Sensors On CFR-PEEK For Smart Orthopedic Implants", 2022 IEEE 35TH INTERNATIONAL CONFERENCE ON MICRO ELECTRO MECHANICAL SYSTEMS CONFERENCE (MEMS), IEEE, 9 January 2022 (2022-01-09), pages 794 - 797, XP034082558, DOI: 10.1109/MEMS51670.2022.9699628
- HENRIQUES BRUNO, FABRIS DOUGLAS, MESQUITA-GUIMARÃES JOANA, SOUSA ANNE C., HAMMES NATHALIA, SOUZA JÚLIO C.M., SILVA FILIPE S., FRED: "Influence of laser structuring of PEEK, PEEK-GF30 and PEEK-CF30 surfaces on the shear bond strength to a resin cement", JOURNAL OF THE MECHANICAL BEHAVIOR OF BIOMEDICAL MATERIALS, ELSEVIER, AMSTERDAM, NL, vol. 84, 1 August 2018 (2018-08-01), AMSTERDAM, NL, pages 225 - 234, XP093075344, ISSN: 1751-6161, DOI: 10.1016/j.jmbbm.2018.05.008

## Description

### TECHNICAL FIELD

The present disclosure relates to the field of wireless sensing, particularly to a carbon-fiber-reinforced polyetheretherketone (CFR-PEEK) orthopedic implant and a preparation method therefor, and a wireless sensing device.

### BACKGROUND

Monitoring of postoperative healing in orthopedics has always been an important and difficult research topic. In the process of postoperative rehabilitation, different weight bearing and activity patterns will affect a force suffered by and a slight motion of a fracture part, thereby having an irreversible influence on the healing of the part.

Currently, the most commonly used postoperative observation method is an X-ray plain film or X-Ray Computer Tomography (CT). However, the ionizing radiation of X-rays and the inconvenience of follow-up visits result in a low sampling frequency for this method, and fail to meet a demand on convenient monitoring at any time. DU YA-WEI ET AL discloses a study titled "Physical modification of polyetheretherketone for orthopedic implants", published in Frontiers of Materials Science, vol. 8, no. 4, pages 313-324 (2014). Henriques Bruno et al. discloses a study titled "Influence of laser structuring of PEEK, PEEK-GF30 and PEEK-CF30 surfaces on the shear bond strength to a resin cement", published in Journal of the Mechanical Behavior of Biomedical Materials, vol. 84, pages 225-234 (2018). [0009] Patent application CN101291634A discloses an instrumented orthopedic implant including a sensor and associated electronic components located within a groove on the implant's outer surface.

Therefore, if real-time detection and continuous monitoring of an orthopedic injury part can be performed, surgery and postoperative rehabilitation can be guided better, thereby a complication can be found and treated more timely.

### SUMMARY

The present disclosure is made based on the inventor's discovery and understanding of the following facts and issues. The invention is set out in the appended set of claims. Any references to embodiments not falling within the scope of the independent claims are to be interpreted as examples useful for understanding the invention.

As described above, an X-ray plain film or X-Ray CT cannot conveniently detect healing of an injured part at any time. A method of additionally adhering a sensor to an orthopedic implant and detecting the healing of an injured part by using an extracorporeal antenna to detect signals has obvious shortcomings: the additionally adhered sensor is adhered to a metal implant by a glue, after working in a body for a long time, the viscosity of the adhesion layer (i.e., the glue layer) decreases, which leads to a poor adherence of the sensor to the metal implant matrix, resulting in a stiffness mismatch problem, which consequently leads to a decrease in the capacitance C and the inductance L of the whole system. According to the resonant frequency calculation formula $RRF = 1 / \left(2π\sqrt{L ⋅ C}\right)$, when the capacitance and the inductance decrease, the resonant frequency will increase, making the resonant response frequency of the sensor itself high, which consequently affects a difficulty of in-vitro detecting and the wireless-sensing-signal receiving, meanwhile, the sensitivity of the sensor will also be greatly reduced, which is not conducive to real-time detection and continuous monitoring of the injured part.

Carbon fiber reinforced polyether-ether-ketone (CFR-PEEK) has comprehensive performance such as excellent mechanical performance, chemical erosion resistance performance and biological compatibility, etc. Through a large number of experimental studies, the inventor found that a patterned carbonization layer can be formed by performing surface carbonization of the CFR-PEEK, the carbonization layer can be used to sense a mechanical change, temperature change or chemical change of an injured part in a body, and these changes can be transferred to an external terminal through signal conversion and transmission, so that healing of the injured part can be known via signals obtained from the terminal.

In view of this, it is one aspect of the present disclosure to provide a CFR-PEEK orthopedic implant, including a CFR-PEEK matrix and a patterned carbonization layer. The patterned carbonization layer is formed by performing in-situ carbonization of the CFR-PEEK matrix. Thus, the patterned carbonization layer formed in situ on the CFR-PEEK matrix may be used to sense a mechanical signal, temperature signal or chemical signal, and may be used in conjunction with an in-vitro probe or an in-vivo signal transduction device to detect and monitor the healing of an injured part in real time, so that a complication can be found in time, and surgery and postoperative rehabilitation can be guided better.

According to embodiments of the present disclosure, the CFR-PEEK orthopedic implant further includes a circuit structure which is provided near the patterned carbonization layer. The circuit structure includes a Wheatstone bridge circuit, an ADC module and a Bluetooth module. The circuit structure receives a resistance signal of the patterned carbonization layer, the resistance signal is converted into a voltage signal through the Wheatstone bridge circuit and is amplified to become an amplified signal, and the amplified signal is performed with analog-to-digital conversion through the ADC module and then is transmitted out through the Bluetooth module.

According to embodiments of the present disclosure, the patterned carbonization layer satisfies at least one of the following conditions: the patterned carbonization layer has a slender strip shape and is used to feed back a mechanical signal; the patterned carbonization layer is a curved connection structure with a plurality of slender lines and is used to feed back a temperature signal; and the patterned carbonization layer is square and is used to feed back a chemical signal. Thus, patterned carbonization layers having different shapes can be used to feed back different signals, which can reflect healing of an injured part more comprehensively.

It is another aspect of the present disclosure to provide a method for preparing a CFR-PEEK orthopedic implant, including: providing a CFR-PEEK matrix; and performing in-situ carbonization of a surface of the CFR-PEEK matrix, to form a patterned carbonization layer. Thus, the patterned carbonization layer may be formed by using in-situ carbonization, the CFR-PEEK orthopedic implant is used to treat fractures, etc., and the patterned carbonization layer formed by in-situ carbonization may be used to sense a mechanical signal, temperature signal, chemical signal of an injured part, which is conducive to real-time detection and monitoring of the injured part, timely detection of a complication, and better guidance for surgery and postoperative recovery.

According to embodiments of the present disclosure, the patterned carbonization layer satisfies at least one of the following conditions: the patterned carbonization layer has a slender strip shape and is used to feed back a mechanical signal; the patterned carbonization layer is a curved connection structure with a plurality of slender lines and is used to feed back a temperature signal; and the patterned carbonization layer is square and is used to feed back a chemical signal.

According to embodiments of the present disclosure, the in-situ carbonization is performed by laser irradiation.

According to embodiments of the present disclosure, the laser irradiation adopts ultraviolet nanosecond laser, and adopts a power of 5 W-10 W, a repetition frequency of 40 kHz-100 kHz, a scanning speed of 20-110 mm/s, and a defocusing amount of 2-10 mm.

It is still another aspect of the present disclosure to provide a wireless sensing device, including the aforementioned CFR-PEEK orthopedic implant, and the CFR-PEEK orthopedic implant is used to be implanted into a human body or an animal body. Thus, the wireless sensing device has all the features and advantages of the aforementioned CFR-PEEK orthopedic implant, which are not repeated here. In general, the wireless sensing device can detect in real time and continuously monitor the healing of the injured part, and can better guide surgery and postoperative healing.

According to embodiments of the present disclosure, the CFR-PEEK orthopedic implant further includes a circuit structure which is provided near the patterned carbonization layer. The circuit structure includes a Wheatstone bridge circuit, an ADC module and a Bluetooth module. The circuit structure receives a resistance signal of the patterned carbonization layer, the resistance signal is converted into a voltage signal through the Wheatstone bridge circuit and is amplified to become an amplified signal, and the amplified signal is performed with analog-to-digital conversion through the ADC module to be converted into a digital voltage signal, the digital voltage signal is then transmitted out through the Bluetooth module. The wireless sensing device further includes a mobile terminal which is used to receive a digital voltage signal transmitted by the Bluetooth module, and convert the digital voltage signal into the resistance signal and display it.

According to embodiments of the present disclosure, a wireless sensing device includes the aforementioned CFR-PEEK orthopedic implant, and the CFR-PEEK orthopedic implant is used to be implanted into a human body or an animal body. The wireless sensing device further includes an in-vitro probe and a network analyzer. The in-vitro probe releases a first alternating electromagnetic field, after the patterned carbonization layer senses a mechanical signal, a temperature signal or a chemical signal, a resonant frequency of the patterned carbonization layer will change, and the patterned carbonization layer generates an induced current under the first alternating electromagnetic field to generate a second alternating electromagnetic field, the second alternating electromagnetic field causes the first alternating electromagnetic field to change, the in-vitro probe senses a change in the first alternating electromagnetic field and transmits it to the network analyzer, and the network analyzer converts the change of the first alternating electromagnetic field into an alternating current signal and converts the alternating current signal into an offset of a peak value of the resonant frequency and displays the offset, so as to obtain information on the mechanical signal, chemical signal or temperature signal at an orthopedic implant injury in a human or an animal by analysis.

### BRIEF DESCRIPTION OF DRAWINGS

The above and/or additional aspects and advantages of the present disclosure will become obvious and easily understood from the description of embodiments in conjunction with the following drawings. In the drawings:
FIG. 1 shows a structural schematic diagram of a carbon fiber reinforced polyether-ether-ketone orthopedic implant according to an embodiment of the present disclosure;
FIG. 2 shows a structural schematic diagram of a carbon fiber reinforced polyether-ether-ketone orthopedic implant according to another embodiment of the present disclosure;
FIG. 3 shows a structural schematic diagram of a carbon fiber reinforced polyether-ether-ketone orthopedic implant according to still another embodiment of the present disclosure;
FIG. 4 shows a structural schematic diagram of a carbon fiber reinforced polyether-ether-ketone orthopedic implant according to yet still another embodiment of the present disclosure;
FIG. 5 shows a structural schematic diagram of a wireless sensing device according to an embodiment of the present disclosure;
FIG. 6 shows a structural schematic diagram of a wireless sensing device according to another embodiment of the present disclosure;
FIG. 7 shows a physical drawing of a network analyzer according to an embodiment of the present disclosure; and
FIG. 8 shows a three-point bending test result of a carbon fiber reinforced polyether-ether-ketone orthopedic implant according to an embodiment of the present disclosure.

### DESCRIPTION OF EMBODIMENTS

Embodiments of the present disclosure are described in detail below. It should be noted that the embodiments described below are exemplary and are intended only to explain the present disclosure and cannot be understood as limitations on the present disclosure. It is understood that the invention is solely defined by the scope of the appended claims. If a specific technology or condition is not indicated in an embodiment, technologies or conditions described in the literatures in the art, or product specifications shall be followed. A reagent or an instrument used, in which a manufacturer is not indicated, are conventional products that can be obtained through market purchase.

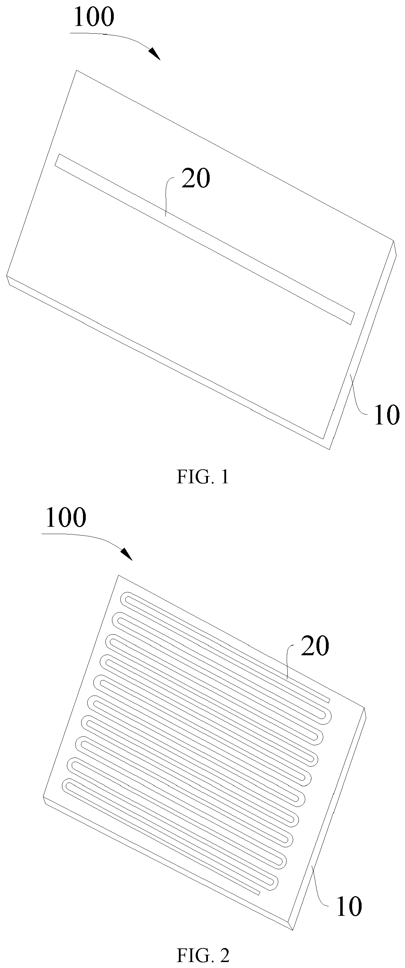

It is one aspect of the present disclosure to provide a carbon fiber reinforced polyether-ether-ketone (CFR-PEEK) orthopedic implant, referring to FIGs. 1 to 3, the CFR-PEEK orthopedic implant 100 includes a CFR-PEEK matrix 10 and a patterned carbonization layer 20. The patterned carbonization layer 20 is formed by performing in-situ carbonization of the CFR-PEEK matrix 10. Thus, the patterned carbonization layer formed in situ on the CFR-PEEK matrix can be used to sense a mechanical signal, temperature signal or chemical signal in a body, and can be used in conjunction with an in-vitro probe or an in-vivo signal transduction device (such as a circuit structure, etc.) to detect and monitor the healing of an injured part in real time, so that a complication can be found in time, and surgery and postoperative rehabilitation can be guided better. In addition, since the patterned carbonization layer is formed on the matrix through in-situ carbonization, the bonding between the patterned carbonization layer and the matrix is very firm, without the problem of weak bonding as the time passes, and thus without the problem of a significant increase in the resonance response frequency.

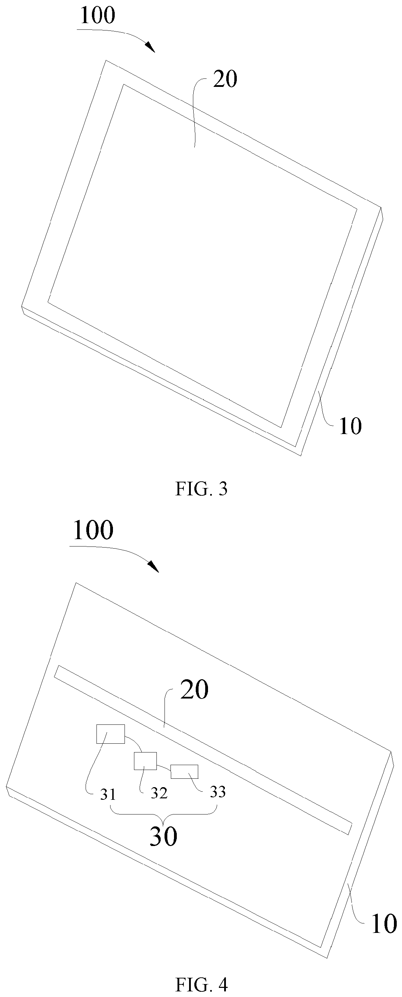

According to the embodiments of the present disclosure, the patterned carbonization layer 20 in the CFR-PEEK orthopedic implant 100 may have different patterns to feed back different signal features. Specifically, according to a specific embodiment of the present disclosure, referring to FIG. 1, the patterned carbonization layer 20 may be in a slender strip shape. The patterned carbonization layer having a slender strip shape is more sensitive to a mechanical signal, and can accurately and timely respond to the mechanical signal, so that the patterned carbonization layer 20 may be used to feed back a mechanical signal, such as a stress, a strain, etc. According to another specific embodiment of the present disclosure, referring to FIG. 2, the patterned carbonization layer 20 may be a curved connection structure with a plurality of slender lines, this structure can sense a subtle change in temperature, so that the patterned carbonization layer 20 may be used to feed back a temperature signal. According to still another specific embodiment of the present disclosure, referring to FIG. 3, the patterned carbonization layer 20 may be square, this square patterned carbonization layer can sense a change in a liquid by absorbing compositions of the liquid, so that the patterned carbonization layer 20 can be used to feed back a chemical signal, such as a change in pH in a body, etc. It should be noted that the above shapes of the patterned carbonization layer in the present disclosure are only for explanation rather than for limiting the present disclosure.

According to embodiments of the present disclosure, referring to FIG. 4, the CFR-PEEK orthopedic implant 100 may further include a circuit structure 30 which is provided near the patterned carbonization layer 20. The circuit structure includes a Wheatstone bridge circuit 31, an ADC (analog-digital conversion) module 32, and a Bluetooth module 33. The patterned carbonization layer 20 senses different signals and transmits them to the circuit structure 20 in the form of a resistance signal. The circuit structure 20 can receive the resistance signal of the patterned carbonization layer 20, and the resistance signal is converted to a voltage signal through the Wheatstone bridge circuit 31 and is amplified to become an amplified signal. Since the amplified signal is a virtual voltage signal and cannot be transmitted through the Bluetooth module, the amplified signal needs to be performed with analog-digital conversion through the ADC module 32 to be converted into a digital voltage signal, and then is transmitted out through the Bluetooth module 33.

It is another aspect of the present disclosure to provide a method for preparing a CFR-PEEK orthopedic implant, including:
S100: providing a CFR-PEEK matrix.

In this step, a CFR-PEEK matrix is provided. The CFR-PEEK is a polyether-ether-ketone composite material with a reinforced carbon fiber, concentrates advantages of a polyether-ether-ketone material and a carbon fiber material, has a light mass, and has excellent mechanical performance, excellent chemical erosion resistance performance and excellent biological compatibility, so that the CFR-PEEK can be used as an orthopedic implant.

S200: performing in-situ carbonization of a surface of the CFR-PEEK matrix.

In this step, a patterned carbonization layer is formed by performing in-situ carbonization of a surface of the CFR-PEEK matrix, so by this simple method, the patterned carbonization layer can be formed on a surface of the CFR-PEEK matrix. The pattered carbonization layer in the CFR-PEEK orthopedic implant can be used to sense a mechanical signal, temperature signal or chemical signal in a body, which is conducive to real-time detection and real-time monitoring of healing of an injured part.

The shapes of the patterned carbonization layer have been described in detail in the preceding text, and are not repeated here.

According to the embodiments of the present disclosure, a specific mode of performing in-situ carbonization of the CFR-PEEK matrix may be laser irradiation. Specifically, laser may be directly irradiated on a surface of the CFR-PEEK, so that a carbonization conductive layer with high conductivity is carbonized on an accurately selected region of the surface of the CFR-PEEK by means of instantaneous high-temperature laser carbonization. The carbonization conductive layer is directly used as an in-situ sensor for sensing a mechanical signal, temperature signal or chemical signal in a body.

According to the embodiments of the present disclosure, laser irradiation may use ultraviolet light, visible light, or infrared light, and may use a pulse width of millisecond, nanosecond, picosecond, or femtosecond, etc., as long as it is capable of carbonizing a required patterned carbonization layer on the surface of the CFR-PEEK. In addition, an energy density of the laser may be changed by changing laser parameters such as an output power, a scanning speed, a repetition rate, defocusing amount of laser, thereby changing the morphology, compositions and resistivity of the patterned carbonization layer. Meanwhile, it is also possible to design a pattern of the carbonization conductive layer (carbonization layer) by designing a laser processing trajectory, to form a sensor having different patterns and applicable for composite signal sensing such as mechanics (pressure, strain, friction, etc.), temperature, chemistry (pH, etc.).

According to some embodiments of the present disclosure, ultraviolet nanosecond laser may be adopted for laser irradiation, an output power of a laser may be 5 W-10 W, for example may be 5 W, 6 W, 7 W, 8 W, 9 W, 10 W, etc., a repetition frequency may be 40 kHz-100 kHz, for example may be 40 kHz, 50 kHz, 60 kHz, 70 kHz, 80 kHz, 90 kHz, 100 kHz, etc., and a scanning speed may be 20-110 mm/s, for example may be 20 mm/s, 30 mm/s, 40 mm/s, 50 mm/s, 60 mm/s, 70 mm/s, 80 mm/s, 90 mm/s, 100 mm/s, 110 mm/s, etc., and a defocusing amount may be 2-10 mm, for example may be 2 mm, 3 mm, 4 mm, 5 mm, 6 mm, 7 mm, 8 mm, 9 mm, 10 mm, etc. Thus, an energy density of irradiating on the surface of the CFR-PEEK matrix may be greater than 0.83 J/mm², thereby forming the carbonization layer on the surface of the CFR-PEEK matrix.

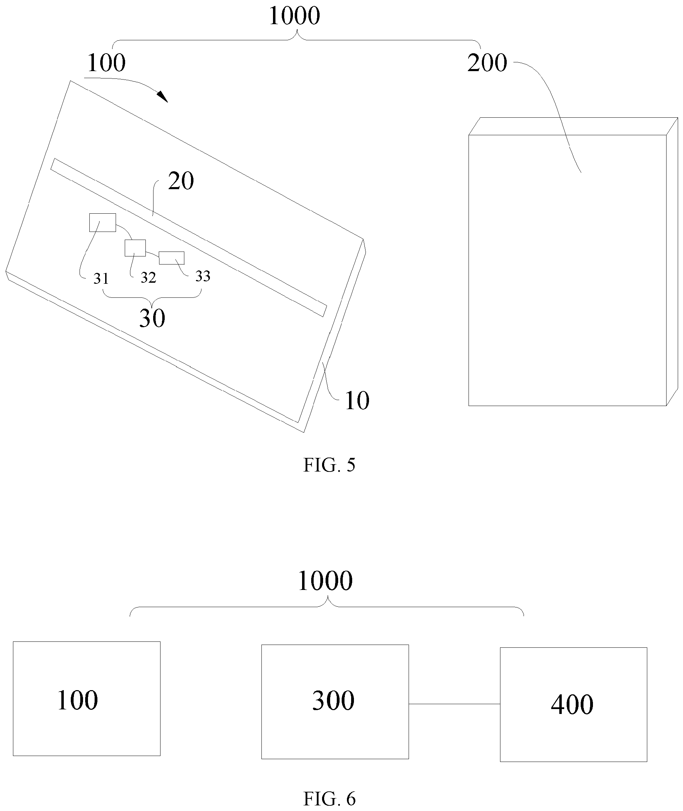

It is still another aspect of the present disclosure to provide a wireless sensing device, referring to FIG. 5 and FIG. 6, the wireless sensing device 1000 includes the aforementioned CFR-PEEK orthopedic implant 100 that is used to be implanted into a human body or an animal body. The wireless sensing device has all the features and advantages of the aforementioned CFR-PEEK orthopedic implant, which are not repeated here. In general, the wireless sensing device may be used for real-time detection and real-time monitoring of the healing of an injured part, can timely feed back a mechanical signal, temperature signal and chemical signal of the injured part, which is convenient for follow-up, and can be used to guide surgery and postoperative rehabilitation treatment.

According to some embodiments of the present disclosure, referring to FIG. 5, the CFR-PEEK orthopedic implant 100 further includes a circuit structure 30 which is provided near the patterned carbonization layer 20. The circuit structure 30 includes a Wheatstone bridge circuit 31, an ADC module 32, and a Bluetooth module 33. The circuit structure 30 receives a resistance signal of the patterned carbonization layer 20, the resistance signal is converted into a voltage signal through the Wheatstone bridge circuit 31 and is amplified to become an amplified signal, and the amplified signal is performed with analog-to-digital conversion through the ADC module 32 to be converted into a digital voltage signal, the digital voltage signal is then transmitted out through the Bluetooth module 32. Referring to FIG. 5, the wireless sensing device 1000 further includes a mobile terminal 200 which is used to receive a digital voltage signal transmitted by the Bluetooth module 33, and convert the digital voltage signal into a resistance signal and display the resistance signal. It should be noted that the mobile terminal may be a device or apparatus with a display function, such as a mobile phone or a computer, etc.

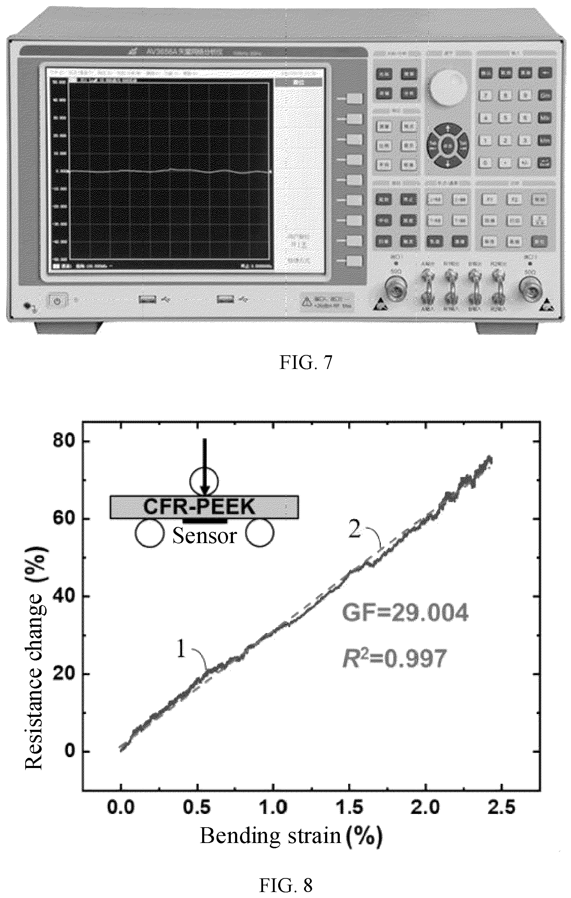

According to some embodiments of the present disclosure, referring to FIG. 6, the wireless sensing device 1000 comprises a CFR-PEEK orthopedic implant 100, and further includes an in-vitro probe 300 and a network analyzer 400. FIG. 7 is a physical drawing of the network analyzer 400, which includes a display screen and a plurality of adjustment buttons, etc. The working principle of the wireless sensing device 1000 is provided as follows:

The in-vitro probe 300 releases a first alternating electromagnetic field; after the patterned carbonization layer 20 senses a mechanical signal, a temperature signal or a chemical signal, a resonant frequency of the patterned carbonization layer 20 will change, and the patterned carbonization layer 20 generates an induced current under the first alternating electromagnetic field to generate a second alternating electromagnetic field, the second alternating electromagnetic field will cause the first alternating electromagnetic field to change; the in-vitro probe 300 senses a change in the first alternating electromagnetic field and transmits it to the network analyzer 400; the network analyzer 400 converts the change of the first alternating electromagnetic field into an alternating current signal, and converts the alternating current signal into an offset of a peak value of the resonant frequency and displays the offset, so as to obtain change information on the mechanical signal, chemical signal or temperature signal at an orthopedic implant injury in a human or an animal by analysis.

The solution of the present disclosure is described via the following specific embodiment.

### Embodiment 1

Ultraviolet nanosecond laser is used to irradiate a surface of a CFR-PEEK (CFR-PEEK) matrix, the ultraviolet nanosecond laser used has a wavelength of 355 nm and a pulse width of 25 ns, and a laser used has an output power of 5.5 W, a repetition rate of 40 kHz, a scanning speed of 60 mm/s, and a defocusing amount of 2 mm, so that a patterned carbonization layer is formed on the surface of the CFR-PEEK matrix to obtain a CFR-PEEK orthopedic implant.

A three-point bending text is carried out on the CFR-PEEK orthopedic implant, and the text result is shown FIG. 8, in which the ordinate resistance change refers to a ratio of an absolute value of a resistance change to an initial resistance value, expressed as a percentage, the initial resistance value refers to a resistance value measured when no bending strain occurs, and the absolute value of the resistance change refers to an absolute value of a difference value between a resistance value measured after bending strain occurs and the initial resistance value. The curve 1 is a curve in which a resistance change obtained by an actual test varies with bending strain, and the dashed line 2 is obtained by origin software fitting. Upon calculation, within a working strain range of 0-2.5%, the orthopedic implant has a higher degree of linearity (R²=0.997), the degree of linearity is close to 1, indicating that use of this orthopedic implant to sense a signal change has good reliability and the repeatability is higher. Meanwhile, the test shows that this orthopedic implant also has higher sensitivity (Gauge Factor (GF), the sensitivity is 29.0074, the sensitivity is higher, indicating that this orthopedic implant is more sensitive to a signal change and can be used to detect a tiny signal change.

Through the above experiment, it can be known that the patterned carbonization layer in the CFR-PEEK orthopedic implant can better feed back a mechanical signal, this orthopedic implant can be used in a human or animal body, and can be used in conjunction with an in-vitro probe or an in-vivo peripheral circuit to achieve real-time detection and real-time monitoring of the healing of an injured part.

In the present disclosure, the healing of the injury refers to healing of an injury at a fracture part. During a gradual healing process of the injured part, the bending strain sensed by the patterned carbonization layer will gradually decrease. Under normal circumstances, when the healing is successful, the bending strain sensed by the patterned carbonization layer will be about 2%, at this moment, a corresponding resistance change in FIG. 8 is about 60%. That is, if the resistance change sensed by the patterned carbonization layer is greater and gradually decreases as time passes, it can be considered that the injured part is gradually healing; however, if the resistance change suddenly increases, it can be considered that inflammation or other complications occur in the injured part, corresponding treatment measures may be taken in time; and if the resistance change of the patterned carbonization layer is about 60% or less, it can be considered that the injured part is healed successfully. For a temperature signal, if a temperature sensed by the patterned carbonization layer is slightly higher than 37 °C, it can be considered that the injured part has not completely healed, while if the temperature sensed by the patterned carbonization layer is 37 °C or slightly lower than 37 °C, it can be considered that the injured part has successfully healed.

In the description of the present Specification, description of reference terms "one embodiment", "another embodiment", "a further embodiment", "a specific embodiment", "another specific embodiment", "a further specific embodiment" and "some embodiments", etc. means that a specific feature, structure, material or characteristic described in conjunction with this example is included in at least one example of the present disclosure. In the present Specification, schematic representations of the above terms are not necessary to aim at the same examples. And, the described specific feature, structure, material or characteristic may be combined in a suitable manner in any one or more examples. In addition, without contradicting each other, persons skilled in the art may incorporate and combine different examples described in the present Specification as well as the features of different examples. In addition, it should be noted that in the present Specification, the terms "first" and "second" are used for the descriptive purpose only and cannot be understood as indicating or implying relative importance or as implicitly indicating the number of the indicated technical features.

Although examples of the present disclosure have been shown and described above, it can be understood that the above examples cannot be understood as limitations on the present disclosure, while the invention is solely defined by the scope of the appended claims.

## Claims

1. A carbon fiber reinforced polyether-ether-ketone, CFR-PEEK, orthopedic implant (100), comprising a CFR-PEEK matrix (10) and a patterned carbonization layer (20);
wherein the patterned carbonization layer (20) is a carbonized region of the CFR-PEEK matrix (10);
**characterized in that**
the patterned carbonization layer (20) satisfies at least one of the following conditions:
the patterned carbonization layer (20) has a slender strip shape and is configured to feed back a mechanical signal;
the patterned carbonization layer (20) is a curved connection structure with a plurality of slender lines and is configured to feed back a temperature signal; and
the patterned carbonization layer (20) is square and is configured to feed back a chemical signal.

2. The CFR-PEEK orthopedic implant (100) according to claim 1, further comprising a circuit structure (30) which is provided near the patterned carbonization layer (20), and the circuit structure (30) comprises a Wheatstone bridge circuit (31), an ADC module (32), and a Bluetooth module (33);
the circuit structure (30) is configured to receive a resistance signal of the patterned carbonization layer (20);
the Wheatstone bridge circuit (31) is configured to convert the resistance signal into a voltage signal and amplify the voltage signal to obtain an amplified signal;
the ADC module (32) is configured to perform analog-to-digital conversion on the amplified signal; and
the Bluetooth module (33) is configured to transmit the amplified signal that has undergone analog-to-digital conversion.

3. A method for preparing a CFR-PEEK orthopedic implant (100), comprising:
providing a CFR-PEEK matrix (10); and
performing in-situ carbonization of a surface of the CFR-PEEK matrix (10), to form a patterned carbonization layer (20);
**characterized in that** the patterned carbonization layer (20) satisfies at least one of the following conditions:
the patterned carbonization layer (20) has a slender strip shape and is used to feed back a mechanical signal;
the patterned carbonization layer (20) is a curved connection structure with a plurality of slender lines and is used to feed back a temperature signal; and
the patterned carbonization layer (20) is square and is used to feed back a chemical signal.

4. The method according to claim 3, wherein the in-situ carbonization is performed by laser irradiation.

5. The method according to claim 4, wherein the laser irradiation adopts ultraviolet nanosecond laser, and adopts a power of 5 W-10 W, a repetition frequency of 40 kHz-100 kHz, a scanning speed of 20-110 mm/s, and a defocusing amount of 2-10 mm.

6. A wireless sensing device (1000), comprising a CFR-PEEK orthopedic implant (100) according to claim 1 or 2, the CFR-PEEK orthopedic implant (100) being configured to be implanted into a human body or an animal body.

7. The wireless sensing device (1000) according to claim 6, wherein the CFR-PEEK orthopedic implant (100) further includes a circuit structure (30) provided near the patterned carbonization layer (20);
wherein the circuit structure (30) comprises a Wheatstone bridge circuit (31), an ADC module (32) and a Bluetooth module (33);
the circuit structure (30) is configured to receive a resistance signal of the patterned carbonization layer (20);
the Wheatstone bridge circuit (31) is configured to convert the resistance signal into a voltage signal and amplify the voltage signal to obtain an amplified signal;
the ADC module (32) is configured to perform analog-to-digital conversion on the amplified signal to convert it into a digital voltage signal;
the Bluetooth module (33) is configured to transmit the digital voltage signal; and
the wireless sensing device (1000) further comprises a mobile terminal (200), which is configured to receive a digital voltage signal transmitted by the Bluetooth module (33), convert the digital voltage signal into the resistance signal, and display the resistance signal.

8. The wireless sensing device (1000) according to claim 6, wherein the wireless sensing device (1000) further comprises an in-vitro probe (300) and a network analyzer (400);
the in-vitro probe (300) is configured to release a first alternating electromagnetic field;
the patterned carbonization layer (20) is configured to: change its resonant frequency upon sensing a mechanical signal, a temperature signal, or a chemical signal, and generate an induced current under the first alternating electromagnetic field to produce a second alternating electromagnetic field that causes the first alternating electromagnetic field to change;
the in-vitro probe (300) is configured to sense a change in the first alternating electromagnetic field and transmit it to the network analyzer (400),
the network analyzer (400) is configured to: convert the change of the first alternating electromagnetic field into an alternating current signal, convert the alternating current signal into an offset of a peak value of the resonant frequency, and display the offset, so as to obtain change information on the mechanical signal, chemical signal or temperature signal at an orthopedic implant injury in a human or an animal by analysis.

## Patentansprüche

1. Orthopädisches kohlenstofffaserverstärktes Polyetheretherketon-, CFR-PEEK-, Implantat (100), umfassend eine CFR-PEEK-Matrix (10) und eine strukturierte Karbonisierungsschicht (20);
wobei die strukturierte Karbonisierungsschicht (20) ein karbonisierter Bereich der CFR-PEEK-Matrix (10) ist;
**dadurch gekennzeichnet, dass**
die strukturierte Karbonisierungsschicht (20) mindestens eine der folgenden Bedingungen erfüllt:
die strukturierte Karbonisierungsschicht (20) weist die Form eines schmalen Streifens auf und ist dazu ausgebildet, ein mechanisches Signal rückzumelden;
die strukturierte Karbonisierungsschicht (20) ist eine gekrümmte Verbindungsstruktur mit einer Vielzahl von schmalen Linien und ist dazu ausgebildet, ein Temperatursignal rückzumelden; und
die strukturierte Karbonisierungsschicht (20) ist quadratisch und ist dazu ausgebildet, ein chemisches Signal rückzumelden.

2. Orthopädisches CFR-PEEK-Implantat (100) nach Anspruch 1, weiter umfassend eine Schaltungsstruktur (30), die nahe der strukturierten Karbonisierungsschicht (20) bereitgestellt ist, und die Schaltungsstruktur (30) umfasst eine Wheatstone-Brückenschaltung (31), ein ADC-Modul (32) und ein Bluetooth-Modul (33);
die Schaltungsstruktur (30) ist dazu ausgebildet, ein Widerstandssignal der strukturierten Karbonisierungsschicht (20) zu empfangen;
die Wheatstone-Brückenschaltung (31) ist dazu ausgebildet, das Widerstandssignal in ein Spannungssignal umzuwandeln und das Spannungssignal zu verstärken, um ein verstärktes Signal zu erhalten;
das ADC-Modul (32) ist dazu ausgebildet, an dem verstärkten Signal eine Analog-Digital-Wandlung durchzuführen; und
das Bluetooth-Modul (33) ist dazu ausgebildet, das einer Analog-Digital-Wandlung unterzogene verstärkte Signal zu übertragen.

3. Verfahren zum Herstellen eines orthopädischen CFR-PEEK-Implantats (100), umfassend:
Bereitstellen einer CFR-PEEK-Matrix (10); und
Durchführen einer In-situ-Karbonisierung einer Oberfläche der CFR-PEEK-Matrix (10), um eine strukturierte Karbonisierungsschicht (20) zu bilden;
**dadurch gekennzeichnet, dass** die strukturierte Karbonisierungsschicht (20) mindestens eine der folgenden Bedingungen erfüllt:
die strukturierte Karbonisierungsschicht (20) weist die Form eines schmalen Streifens auf und wird dazu verwendet, ein mechanisches Signal rückzumelden;
die strukturierte Karbonisierungsschicht (20) ist eine gekrümmte Verbindungsstruktur mit einer Vielzahl von schmalen Linien und wird dazu verwendet, ein Temperatursignal rückzumelden; und
die strukturierte Karbonisierungsschicht (20) ist quadratisch und wird dazu verwendet, ein chemisches Signal rückzumelden.

4. Verfahren nach Anspruch 3, wobei die In-situ-Karbonisierung durch Laserbestrahlung durchgeführt wird.

5. Verfahren nach Anspruch 4, wobei die Laserbestrahlung einen Ultraviolett-Nanosekundenlaser verwendet, und eine Leistung von 5 W-10 W, eine Wiederholfrequenz von 40 kHz-100 kHz, eine Scangeschwindigkeit von 20-110 mm/s und einen Defokussierungsabstand von 2-10 mm verwendet.

6. Drahtlose Erfassungsvorrichtung (1000), umfassend ein orthopädisches CFR-PEEK-Implantat (100) nach Anspruch 1 oder 2, wobei das orthopädische CFR-PEEK-Implantat (100) dazu ausgebildet ist, in einen menschlichen Körper oder einen tierischen Körper implantiert zu werden.

7. Drahtlose Erfassungsvorrichtung (1000) nach Anspruch 6, wobei das orthopädische CFR-PEEK-Implantat (100) weiter eine nahe der strukturierten Karbonisierungsschicht (20) bereitgestellte Schaltungsstruktur (30) einschließt;
wobei die Schaltungsstruktur (30) eine Wheatstone-Brückenschaltung (31), ein ADC-Modul (32) und ein Bluetooth-Modul (33) umfasst;
die Schaltungsstruktur (30) dazu ausgebildet ist, ein Widerstandssignal der strukturierten Karbonisierungsschicht (20) zu empfangen;
die Wheatstone-Brückenschaltung (31) dazu ausgebildet ist, das Widerstandssignal in ein Spannungssignal umzuwandeln und das Spannungssignal zu verstärken, um ein verstärktes Signal zu erhalten;
das ADC-Modul (32) dazu ausgebildet ist, an dem verstärkten Signal eine Analog-Digital-Wandlung durchzuführen, um es in ein digitales Spannungssignal umzuwandeln;
das Bluetooth-Modul (33) dazu ausgebildet ist, das digitale Spannungssignal zu übertragen; und
die drahtlose Erfassungsvorrichtung (1000) weiter ein mobiles Endgerät (200) umfasst, das dazu ausgebildet ist, ein durch das Bluetooth-Modul (33) übertragenes digitales Spannungssignal zu empfangen, das digitale Spannungssignal in das Widerstandssignal umzuwandeln und das Widerstandssignal anzuzeigen.

8. Drahtlose Erfassungsvorrichtung (1000) nach Anspruch 6, wobei die drahtlose Erfassungsvorrichtung (1000) weiter eine In-vitro-Sonde (300) und einen Netzwerkanalysator (400) umfasst;
die In-vitro-Sonde (300) dazu ausgebildet ist, ein erstes wechselndes elektromagnetisches Feld freizusetzen;
die strukturierte Karbonisierungsschicht (20) dazu ausgebildet ist: ihre Resonanzfrequenz bei der Erfassung eines mechanischen Signals, eines Temperatursignals oder eines chemischen Signals zu ändern und unter dem ersten wechselnden elektromagnetischen Feld einen induzierten Strom zu erzeugen, um ein zweites wechselndes elektromagnetisches Feld zu erzeugen, das bewirkt, dass sich das erste wechselnde elektromagnetische Feld ändert;
die In-vitro-Sonde (300) dazu ausgebildet ist, eine Änderung des ersten wechselnden elektromagnetischen Feldes zu erfassen und sie an den Netzwerkanalysator (400) zu übertragen,
der Netzwerkanalysator (400) dazu ausgebildet ist: die Änderung des ersten wechselnden elektromagnetischen Feldes in ein Wechselstromsignal umzuwandeln, das Wechselstromsignal in einen Offset eines Spitzenwerts der Resonanzfrequenz umzuwandeln und den Offset anzuzeigen, um durch Analyse Änderungsinformationen über das mechanische Signal, das chemische Signal oder das Temperatursignal bei einer Verletzung eines orthopädischen Implantats in einem Menschen oder einem Tier zu erhalten.

## Revendications

1. Implant orthopédique (100) en polyéther-éther-cétone renforcé de fibres de carbone, CFR-PEEK, comprenant une matrice CFR-PEEK (10) et une couche de carbonisation structurée (20) ;
dans lequel la couche de carbonisation structurée (20) est une zone carbonisée de la matrice CFR-PEEK (10) ;
**caractérisé en ce que**
la couche de carbonisation structurée (20) satisfait à au moins une des conditions suivantes :
la couche de carbonisation structurée (20) présente une forme de bande étroite et est configurée pour renvoyer un signal mécanique ;
la couche de carbonisation structurée (20) est une structure de connexion courbe avec une pluralité de lignes fines et est configurée pour renvoyer un signal de température ; et
la couche de carbonisation structurée (20) est carrée et est configurée pour renvoyer un signal chimique.

2. Implant orthopédique (100) en CFR-PEEK selon la revendication 1, comprenant en outre une structure de circuit (30) qui est située à proximité de la couche de carbonisation structurée (20), et la structure de circuit (30) comprend un circuit en pont de Wheatstone (31), un module CAN (32), et un module Bluetooth (33) ;
la structure de circuit (30) est configurée pour recevoir un signal de résistance de la couche de carbonisation structurée (20) ;
le circuit en pont de Wheatstone (31) est configuré pour convertir le signal de résistance en un signal de tension et amplifier le signal de tension de manière à obtenir un signal amplifié ;
le module CAN (32) est configuré pour effectuer une conversion analogique-numérique du signal amplifié ; et
le module Bluetooth (33) est configuré pour transmettre le signal amplifié qui a subi une conversion analogique-numérique.

3. Procédé de préparation d'un implant orthopédique (100) en CFR-PEEK, comprenant :
la fourniture d'une matrice CFR-PEEK (10) ; et
la réalisation d'une carbonisation in situ d'une surface de la matrice CFR-PEEK (10), pour former une couche de carbonisation structurée (20) ;
**caractérisé en ce que** la couche de carbonisation structurée (20) satisfait à au moins une des conditions suivantes :
la couche de carbonisation structurée (20) présente une forme de bande étroite et est utilisée pour renvoyer un signal mécanique ;
la couche de carbonisation structurée (20) est une structure de connexion courbe avec une pluralité de lignes fines et est utilisée pour renvoyer un signal de température ; et
la couche de carbonisation structurée (20) est carrée et est utilisée pour renvoyer un signal chimique.

4. Procédé selon la revendication 3, dans lequel la carbonisation in situ est réalisée par irradiation laser.

5. Procédé selon la revendication 4, dans lequel l'irradiation laser utilise un laser ultraviolet nanoseconde, et utilise une puissance allant de 5 W à 10 W, une fréquence de répétition allant de 40 kHz à 100 kHz, une vitesse de balayage allant de 20 à 110 mm/s, et une distance de défocalisation allant de 2 à 10 mm.

6. Dispositif de détection sans fil (1000), comprenant un implant orthopédique (100) en CFR-PEEK selon la revendication 1 ou la revendication 2, l'implant orthopédique (100) en CFR-PEEK étant configuré pour être implanté dans un corps humain ou un corps animal.

7. Dispositif de détection sans fil (1000) selon la revendication 6, dans lequel l'implant orthopédique (100) en CFR-PEEK comprend en outre une structure de circuit (30) disposée à proximité de la couche de carbonisation structurée (20) ;
dans lequel la structure de circuit (30) comprend un circuit en pont de Wheatstone (31), un module CAN (32) et un module Bluetooth (33) ;
la structure de circuit (30) est configurée pour recevoir un signal de résistance de la couche de carbonisation structurée (20) ;
le circuit en pont de Wheatstone (31) est configuré pour convertir le signal de résistance en un signal de tension et amplifier le signal de tension de manière à obtenir un signal amplifié ;
le module CAN (32) est configuré pour effectuer une conversion analogique-numérique sur le signal amplifié afin de le convertir en un signal de tension numérique ;
le module Bluetooth (33) est configuré pour transmettre le signal de tension numérique ; et
le dispositif de détection sans fil (1000) comprend en outre un terminal mobile (200), qui est configuré pour recevoir un signal de tension numérique transmis par le module Bluetooth (33), convertir le signal de tension numérique en signal de résistance, et afficher le signal de résistance.

8. Dispositif de détection sans fil (1000) selon la revendication 6, dans lequel le dispositif de détection sans fil (1000) comprend en outre une sonde in vitro (300) et un analyseur de réseau (400) ;
la sonde in vitro (300) est configurée pour générer un premier champ électromagnétique alternatif ;
la couche de carbonisation structurée (20) est configurée pour : modifier sa fréquence de résonance lors de la détection d'un signal mécanique, d'un signal de température, ou d'un signal chimique, et générer un courant induit sous le premier champ électromagnétique alternatif pour produire un second champ électromagnétique alternatif qui amène le premier champ électromagnétique alternatif à changer ;
la sonde in vitro (300) est configurée pour détecter un changement dans le premier champ électromagnétique alternatif et le transmettre à l'analyseur de réseau (400),
l'analyseur de réseau (400) est configuré pour : convertir le changement du premier champ électromagnétique alternatif en un signal de courant alternatif, convertir le signal de courant alternatif en un décalage d'une valeur de pic de la fréquence de résonance, et afficher le décalage, de manière à obtenir par analyse des informations de changement sur le signal mécanique, le signal chimique ou le signal de température au niveau d'une zone lésée d'implant orthopédique chez un humain ou un animal.
